# EUROPEAN PATENT SPECIFICATION

(11) **EP 2 150 241 B1**
(45) Date of publication and mention of the grant of the patent: **25.01.2012**
(21) Application number: 08758931.3
(22) Date of filing: 02.06.2008
(51) Int. Cl.: A61K 9/20, A61K 31/137

(54) **SUSTAINED RELEASE MICROTABLETS COMPRISING TOLTERODINE TARTRATE**
TOLTERODINTARTRATHALTIGE MIKROTABLETTEN MIT VERZÖGERTER FREISETZUNG
MICROCOMPRIMÉS À LIBÉRATION PROLONGÉE COMPORTANT DU TARTRATE DE TOLTÉRODINE

(30) Priority: 06.05.2008 GR 20080100298
(43) Date of publication of application: 10.02.2010
(73) Proprietor: SPECIFAR S.A., 123 51 Ag. Varvara, Athens (GR)
(72) Inventor: KONTOPANOU, Evanthia, GR-14564 Kifissia Athens (GR); POLITIS, Stavros, GR-19001 Keratea Attiki (GR)
(74) Representative: Roukounas, Dimitrios
(86) International application number: PCT/EP2008/004362
(87) International publication number: WO 2009/135520

(56) References cited:
- EP-A- 1 839 649
- WO-A-03/039553
- WO-A-2005/105036
- WO-A-2007/011131
- WO-A-2007/046590
- PODCZECK F ET AL: "Gastrointestinal transit of model mini-tablet controlled release oral dosage forms in fasted human volunteers" JOURNAL OF PHARMACY AND PHARMACOLOGY, vol. 59, no. 7, July 2007 (2007-07), pages 941-945, XP009110272 ISSN: 0022-3573

## Description

### FIELD OF THE INVENTION

The present invention relates to the field of sustained release microtablets for Tolterodine tartrate.

### BACKGROUND OF THE INVENTION

Tolterodine tartrate is an antimuscarinic agent against overactive bladder, which provides superior efficacy when administered as a once-daily formulation. The typical commercially available compound is the L-Tartrate salt of the API (Active Pharmaceutical Ingredient). Pharmacia's EP1128819 describes the commercial sustained release (SR) tolterodine tartrate formulation, a controlled release bead comprising: (i) a core unit of a substantially water-soluble or water-swellable inert material; (ii) a first layer of a substantially water-insoluble polymer on the core unit; (iii) a second drug containing layer covering the first layer and (iv) a third layer of polymer on the second layer effective for controlled release of the active ingredient, wherein the first layer is adopted to control water penetration into the core. In practise these applications also include a second sealcoat over the drug layer and a sealcoat over the effective controlled release layer.

It is obvious that such formulations exhibit two main disadvantages:

First, they are produced via a complicated process. Second, they are susceptible to aging, a well-known phenomenon in the prior art, when ethylcellulose coatings are applied for sustaining the release of the API. In addition curing (or "further gradual coalescence") is critical for the stabilization of the release characteristics. The process of curing is accelerated by heating, typically at temperatures less than 45°C, or at about 60°C when an HPMC overcoat is applied. Aging is also proven by the different specifications of the commercial Tolterodine Tartrate SR product, concerning the release of the API (3hours:40-70% at time of release, 40-85% at end of self life).

It is obvious that stability of the release profile is probably the most important feature of a sustained release dosage form, as it will provide the promised therapeutic advantages over the relevant immediate release drug product, throughout its self-life. Two different approaches have been described in the prior art in order to address the problems of cores coated with multiple coatings. The first approach deals with the production of pellets via less complicated processes ie by reducing the number of coating layers, (WO2007011131, W02007046590, EP1839649 A1)

The second approach deals with the preparation of sustained release cores that are preferably not coated. Such formulations are advantageous as they are produced via simple manufacturing methods and are free of the problems relevant to curing and stability that have already been mentioned. It should be outlined that these formulations should have a small size, in the region of mini or micro tablets. Micro-tablets are tablets with a diameter equal to, or smaller than, 2-3mm (Lennartz, P., Mielck, J.B., 1998. Minitabletting: improving the compactability of paracetamol powder mixtures. Int. J. Pharm. 173, 75-85.). The production of micro-matrices using a tabletting technique is an attractive alternative to the production of pellets, as simple and predictable production processes are applied, which results in high production yields, dosage forms of defined size and strength and reduced batch to batch variability (Rouge et al., 1997 Rouge, N., Cole, E.T., Doelker, E., Buri, P., 1997. Screening of potentially floating excipients for minitablets. S.T.P. Pharm. Sci. 7, 386-392.). In addition, recent studies (Podczeck, 2007, Gastrointestinal transit of model mini-tablet controlled release oral dosage forms in fasted human volunteers, Journal of Pharmacy and Pharmacology, 59: 941-945, Podczeck et al, 2007, The gastric emptying of food as measured by gamma-scintigraphy and electrical impedance tomography (EIT) and its influence on the gastric emptying of tablets of different dimensions, Journal of Pharmacy and Pharmacology, 59: 1527-1536) show that the use of small (3-3.2mm in diameter) non-disintegrating tablets may delay gastric emptying, a feature that is beneficial for the in vivo behaviour of the dosage form in both fasted and fed conditions.

It is obvious that besides the release profile, the size of the dosage form is one of the most important factors when the micro tablets are designed in order to provide similar in vivo behavior with multiparticulate dosage forms such as pellets.

However the formulation of water-soluble APIs such as tolterodine tartrate in mini or micro tablets is problematic, as the cores are not large enough to support sustained release for more than 8 hours. Two solutions have been suggested towards the achievement of SR tablets incorporating water soluble APIs. The first one combines matrix tablet technology with sustained release coatings. The second utilizes cores of increased size and insoluble or high viscosity polymers resulting in the problems related to the in vivo behavior of these systems when compared with multiparticulate dosage forms. As an example of the first approach, WO2005048979 and WO2005105036 describe Tolterodine Tartrate microtablets that combine sustained release cores and sustained release coatings in order to slow down the release of the water soluble API from matrix tablets. In both cases the critical size of 3mm micro-tablets is marginally approximated. Concerning the second approach, some combinations of soluble and insoluble polymers have been adopted.

Previous trials to formulate Tolterodine tartrate into mini tablets (5mm in diameter and 100mg in weight, 4mm in diameter and 40 or 20mg in weight) have shown the following:
- The release profile can be easily manipulated when the tablet size is moderately large. However such a formulation is not in the region of micro tablets and may not provide a multiparticulate dosage form.
- At different levels and viscosity grades of the matrix soluble polymer (hydrogel) the slope of the release is also significantly affected. This means that the initial release (mainly affected by the surface of the dosage form that is free for release) is not affected in a similar way with the release at later stages of the dissolution trials. This is one of the major difficulties when downsizing, as the initial surface available for release is increased, while, for the same formula, the formation of the consecutive diffusion and erosion layers is not significantly affected.

This behavior shows that further downsizing is difficult when applying the approaches described in the prior art, as controlling the release at the initial stages of the formulation is not as feasible as controlling the release at later stages of the dissolution test.

The effect of lubricants and especially Magnesium Stearate on the release of APIs from matrix type tablets has also been studied in the prior art. It has been found that the increase of Magnesium Stearate should not significantly affect the release of the API from the hydrogel-containing matrix. In detail, although it is well known that Mg Stearate can retard the dissolution from immediate release dosage form, this is not valid for sustained releases dosage forms containing hydrogels. Studies in the prior art conclude that the influence of Mg Stearate is insignificant, when incorporated in the dosage form as a coprecipitate (Khan et al, 1995, Controlled release coprecipitates: formulation considerations Journal of Controlled Release 37 (1995) 131-141) or a lubricant (Rekhi et al, 1999, Identification of critical formulation and processing variables for metoprolol tartrate extended-release (ER) matrix tablets1 Journal of Controlled Release 59 327-342, Nellore et al, 1998, Development of metoprolol tartrate extended-release matrix tablet formulations for regulatory policy consideration Journal of Controlled Release 50 247-256).

It is clear that drugs having relatively high solubility in water, for example an aqueous solubility of about 5 mg/ml or greater, present challenges to the formulator wishing to provide a sustained-release dosage form, especially with uncoated dosage forms that have a size in the mini or microtabet region of less than 3mm.

When attempting the development of such a formulation, further study of the release mechanism may be performed using the modelling of the Peppas model to the data. The empirical equation of the Peppas model, also known as the 'Power low" model has been extensively used in the prior art in order to empirically characterize different release mechanisms, especially when the release mechanism is not well known, or more than one type of release phenomena exist.

### SUMMARY OF THE INVENTION

The present invention provides a sustained release uncoated microtablet comprising Tolterodine tartrate with a diameter smaller than 3mm and more preferably equal or smaller than 2mm, so that it can approximate the in vivo behaviour of multiparticulate systems. The microtablets of the present invention may be formulated into multiparticulate dosage forms such as capsules or tablets. The said micro-tablets are capable of releasing the API in more than 8h and are thus capable of providing a once daily formulation for the said API, due to the pharmacokinetic properties of the specific API. In addition, the microtablets have a diameter of 2-3mm and as a result may provide multiparticulate dosage forms with similar in vivo behavior to the coated spheroids. It is obvious that microtablets are an attractive alternative to the coated spheroids, as they are produced via a simple one step method, and since they are not coated, they are not susceptible to the effect of aging and curing is not necessary, thus it does not affect the performance of the dosage form.

The formulation of the present invention comprises a hydrogel from the group of Hydroxypropylmethyl celluloses (HPMC), a diluent, preferably selected from the group of alginates or inorganic diluents, and Magnesium Stearate, which except from being used as a lubricant, it surprisingly exhibits a predominant effect on the release. Hence, contrary to what has been disclosed in the prior art, it has been unexpectedly found that the lubricant may exhibit a predominant role as a release controlling excipient. The formulation of the present invention allows the preparation of sustained release microtablets comprising Tolterodine tartrate that are capable of releasing the API in a controlled manner, without the use of a coating material, organic solvent or insoluble polymers, while the matrix is in the region of micro tablets (2-3mm in diameter), and thus allows the preparation of multiunit dosage forms.

Ideally the release from the micro tablets as described in the present invention takes place in such a way, that when tested in vitro using a USP type I dissolution apparatus (basket) according to US Pharmacopoeia in phosphate buffer at pH 6.8 the release is not more than 30% of the total quantity of Tolterodine in 1 h, approximately 55% in 3hours and not less than 80% in 7h of measurement in said apparatus.

The release characteristics may be optionally further modified via methods known to the man skilled to the art (such as fully or partially coating the core via spraying or compressing an appropriate material), however the core of each micro-tablet itself is capable of releasing Tolterodine Tartrate gradually in the said desired rate.

The microtablets of the present invention are an attractive alternative compared to coated pellets, as they are produced via a one step compression process and coating is not necessary for controlling the release rate within the desired rate.

The release is basically controlled by rate limiting polymers, however the effect of the lubricant on the release is predominant. In the present invention, the lubricant reduces the amount of the API released at each time interval in an almost uniform way and thus may regulate the release at desired levels.

### BRIEF DESCRIPTION OF THE DRAWINGS

**Figure 1****:** Dissolution profiles of Tolterodine Tartrate from 6,5mg micro tablets, each one of these micro tablets containing 2,2% of Tolterodine Tartrate.
**Figure 2****:** Trace plots concerning the responses %released in 1h when the diluent is Sodium alginate (top figure 2.1) or Bicalcium Phosphate (bottom, figure 2.2). Factors: A=Diluent, B=HPMC K15M, C=HPMC K100M, D=Mg Stearate Reference Blend: A:B:C:D=0.23:0.63:0.07:0.03.
**Figure 3****:** Trace plots concerning the responses %released in 3h when the diluent is Sodium alginate (top, figure 3.1) or Bicalcium Phosphate (bottom, figure 3.2). Factors: A=Diluent, B=HPMC K15M, C=HPMC K100M, D=Mg Stearate Reference Blend: A:B:C:D=0.23:0.63:0.07:0.03.
**Figure 4****:** Trace plots concerning the responses %released in 7h when the diluent is Sodium alginate (Release was completed for all formulations with Bicalcium Phosphate as a diluent and thus no useful plot could be constructed). Factors: A=Diluent, B=HPMC K15M, C=HPMC K100M, D=Mg Stearate Reference Blend: A:B:C:D=0.23:0.63:0.07:0.03.
**Figure 5****:** Trace plots concerning the responses AUC in 3h when the diluent is Sodium alginate (top, figure 5.1) or Bicalcium Phosphate (bottom, figure 5.2). Factors: A=Diluent, B=HPMC K15M, C=HPMC K100M, D=Mg Stearate Reference Blend: A:B:C:D=0.23:0.63:0.07:0.03.
**Figure 6****:** Trace plots concerning the responses AUC in 7h (right) when the diluent is Sodium alginate (top, figure 6.1) or Bicalcium Phosphate (bottom, figure 6.2). Factors: A=Diluent, B=HPMC K15M, C=HPMC K100M, D=Mg Stearate Reference Blend: A:B:C:D=0.23:0.63:0.07:0.03.
**Figure 7****:** Trace plots of the response Peppas exponent (n) when the diluent is Sodium alginate (top, figure 7.1) or Bicalcium Phosphate (bottom, figure 7.2). Factors: A=Diluent, B=HPMC K15M, C=HPMC K100M, D=Mg Stearate Reference Blend: A:B:C:D=0.23:0.63:0.07:0.03.

### DETAILED DESCRIPTION OF THE INVENTION

The present invention provides a sustained release pharmaceutical formulation of Tolterodine Tartrate in the form of uncoated micro tablets. Each micro tablet of the present invention has a diameter between 2-3mm, preferably of 2mm and comprises:

The drug substance, in this case Tolterodine Tartrate in an amount that preferably varies between 0.1-5.0%, more preferably between 1.0-3.0% and even more preferably between 1.5-2.5% by weight per micro tablet

A gelling agent, selected from the group of hydroxypropylmethylcelluloses (HPMC), preferably a HPMC with a viscosity of 4,000-100,000 cp at 2% aqueous solution or a mixture of HPMC hydrogels with such grades. The amount of the gelling agent preferably varies between 55-85% by weight per micro tablet.

A diluent, preferably a swellable diluent or an inorganic diluent, selected from the group of alginates or bicalcium phosphate respectively. The amount of the diluent preferably varies between 10-35% and more preferably between 16-30% by weight per micro tablet.

Magnesium Stearate as a lubricant, in an amount larger than the one proposed by the conventional use of the said excipient, wherein the lubricant is mixed together with the rest of the excipients, and not at the end of mixing as proposed in the prior art. According to the present invention the lubricant plays additionally the role of a rate-controlling component,. The amount of the lubricant preferably varies between 2-4% by weight per micro tablet.

The formulation of the present invention may optionally further contain other excipients known to the man skilled in the art, ie other lubricants such as calcium stearate, magnesium aluminum silicate, stearic acid, zinc stearate, sodium stearyl fumarate, diluents selected from the group consisting of a sugar, a sugar alcohol, a starch, a resin and dibasic calcium phosphate, glidants such as talc and colloidal silica dioxide and binders selected from the group consisting of gelatin, L-HPC, starch, a hydroxypropylmethyl cellulose and a polyvinylpyrrolidone as long as their properties allow them to replace the functional constituents without affecting the performance of the system. The classical excipients used for the preparation of the micro tablets of the present invention should exhibit low solubility in water and be free of disintegrant properties.

The micro tablets can be obtained via either a direct compression process or a wet granulation and compression process. In the case of the direct compression approach, the lubricant is mixed from the start with the rest of the excipients, and not at the end of mixing as proposed in the prior art. In addition, the API, the rate hydrogel, the diluent and the lubricant are mixed together, following a geometric model, during which the API and the lubricant are mixed with an equal amount of hydrogel and diluent mixture, and the new powder mixture is mixed with a larger amount of hydrogel and diluent mixture and so on until a uniform mixture is obtained. After the initial mixing, sieving is performed in order to optimize the dispersion of the constituents in the powder mixture. The wet granulation step may be optionally performed when the diluent is not swellable (such as bicalcium phosphate), and the only purpose of performing such a process step is to enhance the dispersion of the API in the dosage form. In this case the API is mixed with the diluent and the powder mixture is granulated with an aqueous medium. The wet granules are dried and mixed with the extragranular powder mixture comprising mainly the hydrogel and the lubricant. The final powder mixture or powder-granular mixture is then compressed into tablets, preferably using multi-tip punches and dies. The formulation of the present invention allows compression forces of more than 100kN to be applied, without sticking of the microtablets, due to the increased amount of the lubricant.

One preferred embodiment of the present invention is a micro-tablet with similar diameter and height of about 2mm, with a weight of 6.5mg comprising:
- Tolterodine Tartrate, in an amount that varies between 1.5-2.5% by weight per micro tablet.
- A mixture of HPMC K15M and K100M, in an amount that varies between 55-85% by weight per micro tablet.
- A diluent, such as sodium alginate or bicalcium phosphate, in an amount that varies between 16-30% by weight per micro tablet.
- Magnesium Stearate in an amount varying between 2-4% by weight per micro tablet.

The formulation of the present invention is capable of providing a once daily sustained release dosage form for Tolterodine Tartrate, without the use of complicated manufacturing processes and the application of coating materials that need curing and are susceptible to aging.

The following example illustrates the the present invention.

### Example 1

The present example illustrates the formulation of the present invention. In order to be able to reduce the release profile uniformly throughout the dissolution curve the approach of incorporating unusual levels of lubricant was adopted. This approach initially aimed at further improving the ejection of the micro tablets from multi tip punches and dies of 2mm, as the micro tablets would be almost spherical, and typically more than 14-16 micro punches exist per compression station (consisting of multi tip punches and an appropriate die). This shape of the micro tablets results in increased contact of the compressed material with the internal walls of the die and consequently to the need for increased lubrication.

When different levels of the lubricant were utilized it was unexpectedly found that the lubricant may exhibit a predominant role as a release controlling excipient.

In order to fully evaluate the effect of the rate controlling lubricant together with the rate controlling polymers a new D-optimal crossed mixture was set up and executed. The levels of the selected factors are presented in Table I.

| Table I: mixture and process factors of the formulations tested throughout the crossed mixture-process design concerning 2mm microtablets with the use of the lubricant as a release regulator. | | | | |
|---|---|---|---|---|
| Mixture components | Role | | Low level | High level |
| HPMC K15M | Hydrocolloid | | 55% | 70% |
| HPMC K100M | High viscosity Hydrocolloid | | 0% | 15% |
| Diluent | Hydrocolloid or common diluent | | 16% | 30% |
| Mq Stearate | Release regulator and lubricant | | 1% | 4% |

| Fixed components | Role | | Proportion | |
|---|---|---|---|---|
| Tolterodine Tartrate | API | | 2,2% | |
| Col. Silicon Dioxide | Glidant | | 2% | |
| Process parameters (categorical) | | Level 1 | Level 2 | |
| Type of Diluent* | | Sod. Alginate | Bicalcium Phosphate | |

These mixtures were prepared by mixing the API, the rate controlling polymer, the diluent and the lubricant using a geometric model, during which the API and the lubricant are mixed with an equal amount of hydrogel and diluent mixture, and the new powder mixture was mixed with a larger amount of hydrogel and diluent mixture and so on until a uniform mixture is obtained. After the initial mixing, sieving was performed in order to optimize the dispersion of the constituents in the powder mixture. The micro tablets were produced via compression using multi-tip punches and dies, with 16 punches per station, where each punch had a diameter of 2mm. The produced micro tablets had a nominal weight of 6,5mg, in order to provide a 91 mg dosage form that would contain 14 micro tablets for the 2mg strength, and a 182mg dosage form that would contain 28 micro tablets for the 4mg strength. This selection was based on the relevant strength and weight of the originator product (approximately 91mg for the 2mg strength and proportional for the 4mg strength). This approach would undoubtedly provide multiparticulate systems, most probably with similar in vivo behavior with the coated pellets prepared via several layers as they are both high-density forms. An interesting feature of the above design is that it would allow the comparison of the relevant effect of the lubricant and the high viscosity HPMC (K100M) to the release of Tolterodine Tartrate from the micro tablets. Two diluents were tested, one with hydrocolloid properties (Sodium Alginate) and an insoluble inorganic excipient (Bicalcium Phosphate). The formulations of Table II were prepared and tested using a Dissolution Test apparatus I according to the US Pharmacopoeia (baskets).

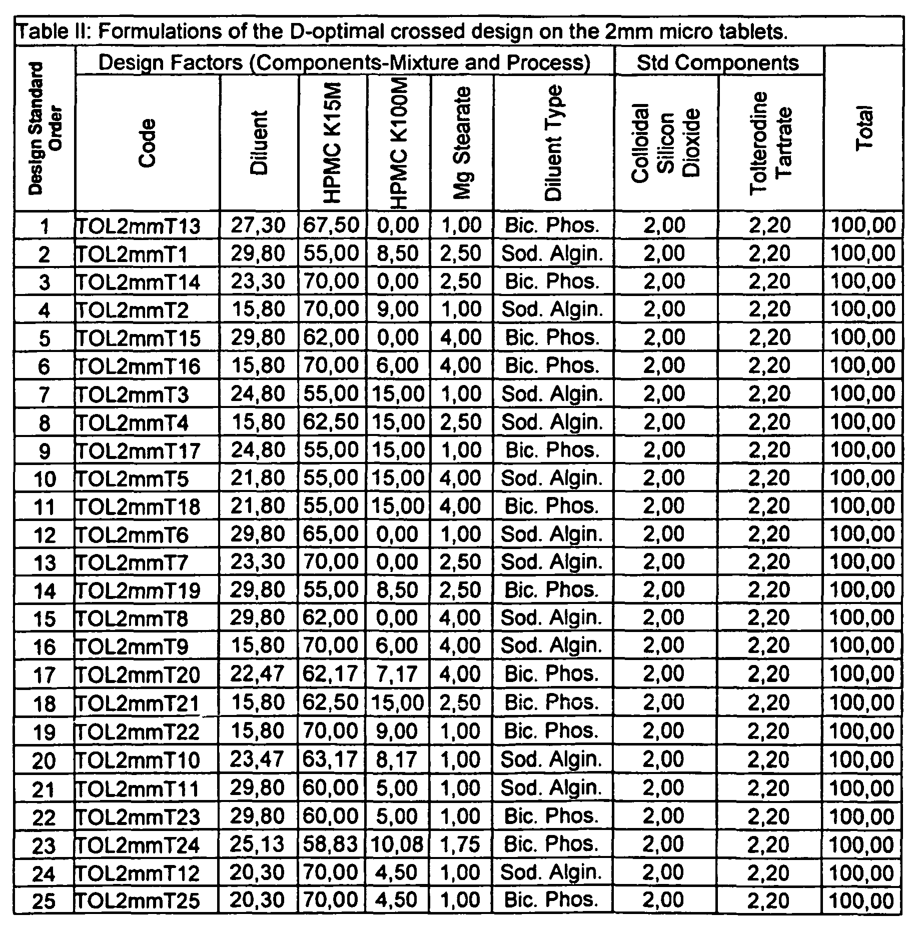

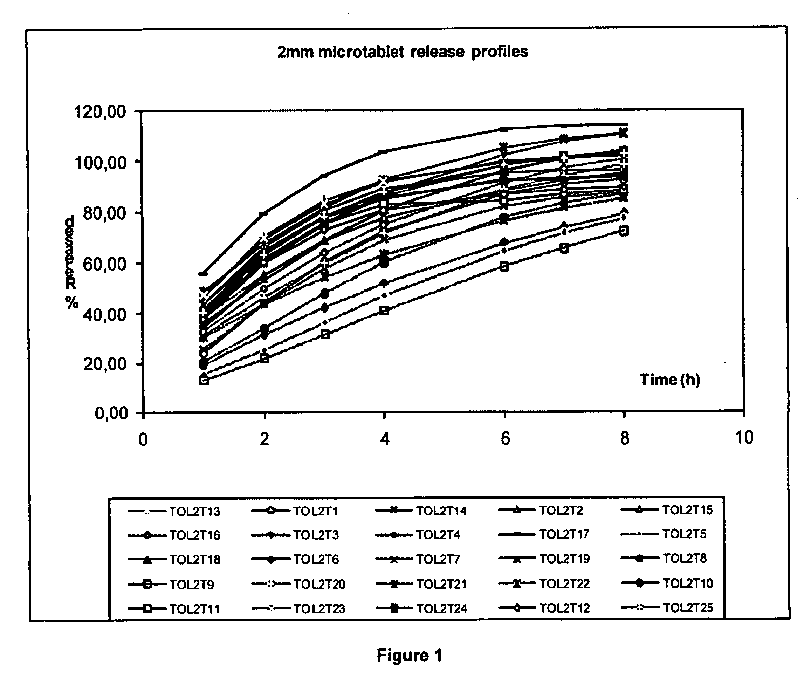

The release profiles from the 2mm tablets are presented in Figure 1.

In order to perform a more formal statistical analysis and test the effect of each of the factors and mainly the relative effect of the lubricant and the high viscosity hydrogel (HPMC K100M), some significant responses were selected, so that they would provide information for the dissolution characteristics throughout the dissolution curve. These responses were the following:
i. %dissolved at the 1^{st} hour of dissolution testing
ii. %dissolved at the 3^{rd} hour of dissolution testing
iii. %dissolved at the 7^{th} hour of dissolution testing
iv. AUC₃ or the Area Under the Curve of the dissolution profile during the first 3hours.
v. AUC₇ or the Area Under the Curve of the dissolution profile during the first 7hours.

The results of Figure 1 are better visualized via graphs known as trace charts. These plots represent the effect of each constituent on a specific quality characteristic, in this case the proportion released and the Area Under the Curve at a certain time periods of the Dissolution profile. The lines represent the effect of changing each mixture component while holding all others in a constant ratio. The response is plotted while moving along an imaginary line from a reference blend to the vertex of the component being incremented. The default reference blend is the centroid of the design. As the amount of this component increases, the amount of all the other components decreases, but their ratio to one another remains constant. When the traces exhibit a large slope, then their effect is significant (Smith, 2005, Experimental Design for Formulation, ASA-SIAM Series on Statistics and Applied Probability, SIAM, Philadelphia, ASA, Alexandria, VA, 2005).

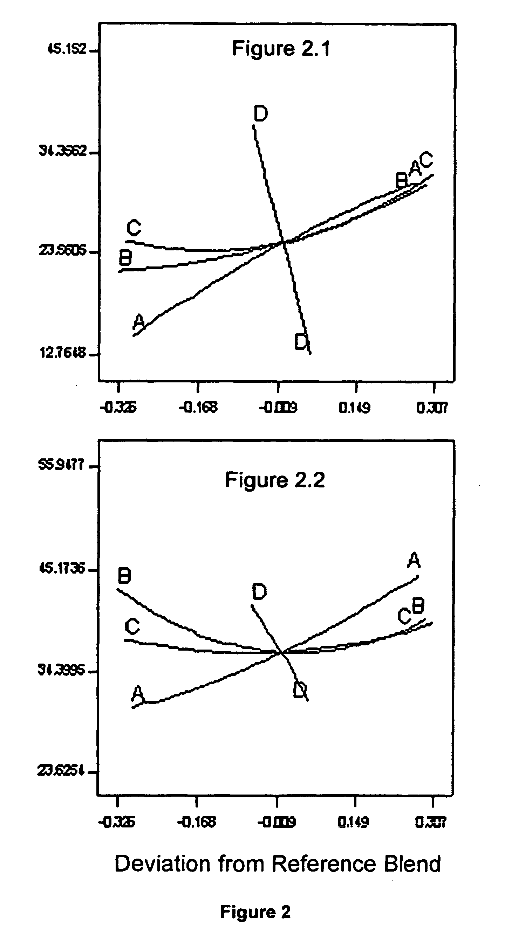

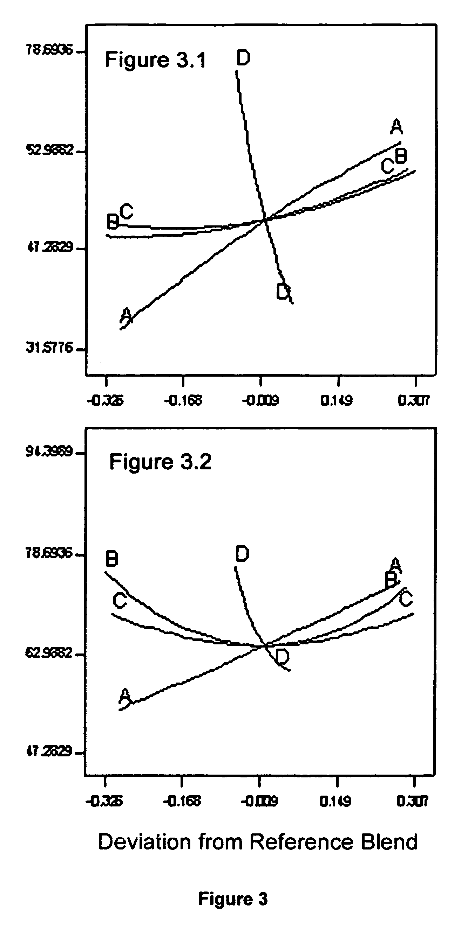

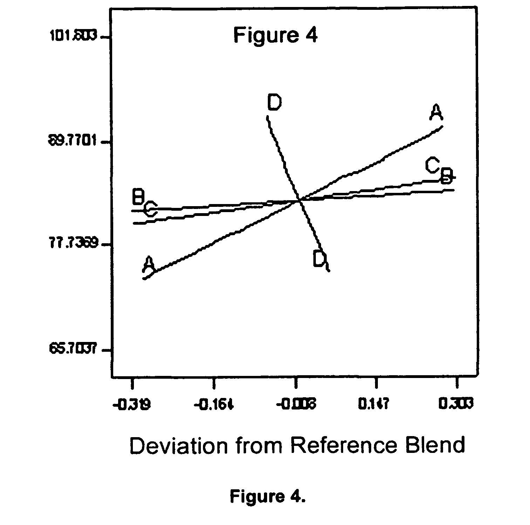

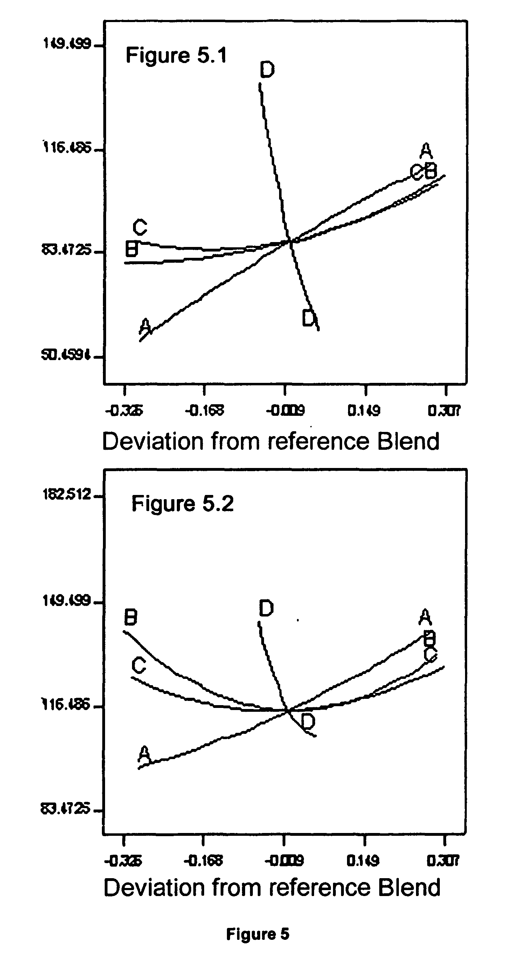

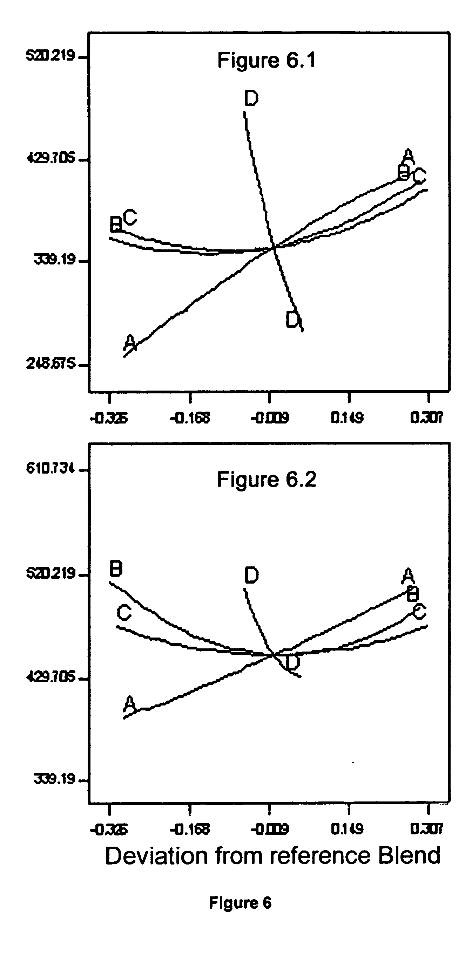

In Figures 2, 3 and 4 the trace Plots (Piepel trace plots) for %released in 1, 3 and 7 hours are presented. In each figure the top diagram refers to the formulations with Sodium Alginate as a diluent (figures 2.1, 3.1 and 4.1) and the bottom diagram to the formulations with Bicalcium Phosphate as a diluent (figures 2.2, 3.2 and 4.2). Similarly in Figures 5 and 6 the trace plots for the responses AUC at 3h and AUC at 7h are presented (AUC3 and AUC7 respectively). In each figure the top diagram refers to the formulations with Sodium Alginate as a diluent (figures 5.1 and 6.1) and the bottom diagram to the formulations with Bicalcium Phosphate as a diluent (figures 5.2 and 5.2).

Through the study of the diagrams in figures 1 to 6 it was surprisingly found that the effect of Mg Stearate is predominant as a rate-controlling constituent, with a greater effect compared to similar (and even larger) quantities of a high viscosity hydrocolloid such as HPMC K100M. This property was beneficial for the preparation of the micro tablets as their thickness is similar to their diameter, thus large ejection forces may develop during the compression process. As a result it is advantageous to be able to assess the behaviour of the formulation when the proportion of the lubricant is increased. In conclusion, with the formulation of the present invention it is feasible to provide sustained release micro tablets with desired release characteristics, with sizes in the region 2-3mm in diameter, more preferably micro tablets with a diameter of 2mm.

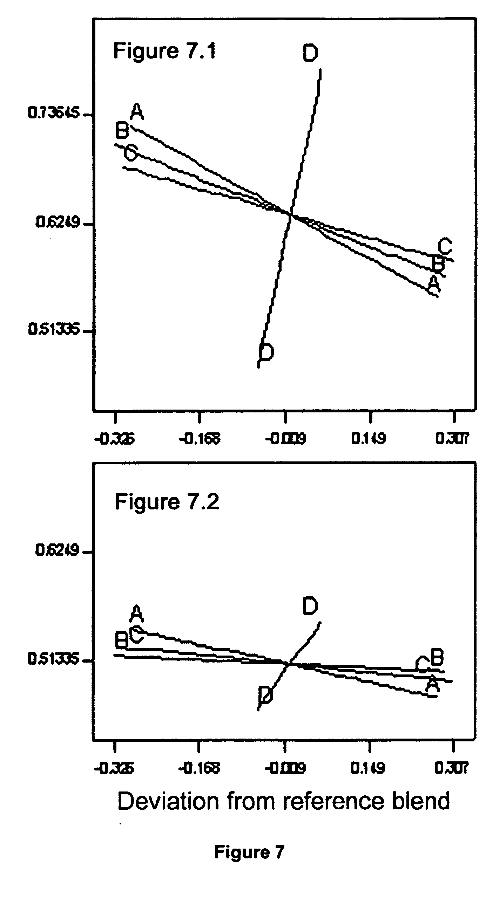

Based on the Peppas model, a trace plot for the Release exponent n was constructed. This plot is presented in figure 7. The top diagram refers to the formulations with Sodium Alginate as a diluent (figure 7.1) and the bottom diagram to the formulations with Bicalcium Phosphate as a diluent (figure 7.2) .

From the plots of figure 7 it is evident that the increase of Mg Stearate may result in an increase of the Peppas exponent n. This means that the mechanism moves towards zero order kinetics (a feature normally expected to be caused by the increase of HPMC K100M). The phenomenon is significantly more intense when Sodium Alginate is used as a diluent. The use of the lubricant according to the formulation of the present invention results in an increase of the Peppas exponent, which is beneficial for the release profile of the API from the micro-tablets.

## Claims

1. A sustained release pharmaceutical composition in the form of uncoated microtablets, wherein said microtablets have a diameter of 2-3mm, preferably of 2mm and comprise tolterodine tartrate, a gelling agent selected from the group of hydroxypropylmethylcelluloses (HPMC), a diluent selected from the group of alginates and 2-4% by weight of magnesium stearate.

2. A sustained release pharmaceutical composition according to claim 1 wherein the gelling agent is a HPMC with a viscosity of 4,000-100,000 cp at 2% aqueous solution or a mixture of HPMC hydrogels with such grades.

3. A sustained release pharmaceutical composition according to anyone of the preceding claims, wherein the amount of tolterodine tartrate varies between 0.1-5.0%, more preferably between 1.0-3.0% and even more preferably between 1.5-2.5% by weight per mini tablet.

4. A sustained release pharmaceutical composition according to anyone of the preceding claims, wherein the amount of the gelling agent varies between 55-85% by weight per mini-tablet.

5. A sustained release pharmaceutical composition according to anyone of the preceding claims, wherein the amount of the diluent varies between 10-35% and preferably between 16-30% by weight per mini tablet.

6. A multiunit dosage form such as capsule or a tablet, comprising the sustained release microtablets of anyone of the preceding claims.

## Patentansprüche

1. Arzneimittel mit verzögerter Freigabe in der Form von unbeschichteten Mikrotabletten, wobei die Mikrotabletten einen Durchmesser von 2-3 mm, vorzugsweise von 2 mm, aufweisen und Tolterodintartrat, ein Geliermittel ausgewählt aus der Gruppe von Hydroxypropylmethylcellulosen (HPMC), ein Verdünnungsmittel ausgewählt aus der Gruppe von Alginaten und 2-4 Gewichtsprozent Magnesiumstearat umfassen.

2. Arzneimittel mit verzögerter Freigabe nach Anspruch 1, wobei das Geliermittel eine HPMC mit einer Viskosität von 4.000-100.000 cp bei einer 2-prozentigen wässrigen Lösung ist oder ein Gemisch von HPMC-Hydrogelen mit solchen Graden.

3. Arzneimittel mit verzögerter Freigabe gemäß irgendeinem der vorausgehenden Ansprüche, wobei der Betrag des Tolterodintartrates zwischen 0,1-5,0 Gewichtsprozent, bevorzugter zwischen 1,0-3,0 Gewichtsprozent und noch bevorzugter zwischen 1,5-2,5 Gewichtsprozent pro Minitablette variiert.

4. Arzneimittel mit verzögerter Freigabe gemäß irgendeinem der vorausgehenden Ansprüche, wobei der Betrag des Geliermittels zwischen 55-85 Gewichtsprozent pro Minitablette variiert.

5. Arzneimittel mit verzögerter Freigabe gemäß irgendeinem der vorausgehenden Ansprüche, wobei der Betrag des Verdünnungsmittels zwischen 10-35 Gewichtsprozent und vorzugsweise zwischen 16-30 Gewichtsprozent pro Minitablette variiert.

6. Eine multipartikuläre Darreichungsform, wie beispielsweise eine Kapsel oder eine Tablette, welche die Mikrotabletten mit verzögerter Freigabe von irgendeinem der vorausgehenden Ansprüche umfasst.

## Revendications

1. Composition pharmaceutique à libération prolongée sous forme de micro-comprimés non enrobés, dans laquelle lesdits micro-comprimés ont un diamètre de 2 à 3 mm, de préférence de 2 mm, et comprennent du tartrate de toltérodine, un agent gélifiant choisi dans le groupe des hydroxypropylméthylcelluloses (HPMC), un diluant choisi dans le groupe des alginates et 2 à 4 % en poids de stéarate de magnésium.

2. Composition pharmaceutique à libération prolongée selon la revendication 1 dans laquelle l'agent gélifiant est une HPMC ayant une viscosité de 4 000 à 100 000 cP dans une solution aqueuse à 2 % ou un mélange d'hydrogels d'HPMC avec lesdites qualités.

3. Composition pharmaceutique à libération prolongée selon l'une quelconque des revendications précédentes dans laquelle la quantité de tartrate de toltérodine varie de 0,1 à 5,0 %, plus préférablement de 1,0 à 3,0 % et de manière préférée entre toutes, de 1,5 à 2,5 % en poids par mini-comprimé.

4. Composition pharmaceutique à libération prolongée selon l'une quelconque des revendications précédentes dans laquelle la quantité d'agent gélifiant varie de 55 à 85 % en poids par mini-comprimé.

5. Composition pharmaceutique à libération prolongée selon l'une quelconque des revendications précédentes, dans laquelle la quantité de diluant varie de 10 à 35 % et de préférence de 16 à 30 % en poids par mini-comprimé.

6. Forme galénique de plusieurs unités, telles qu'une gélule ou un comprimé, comprenant les micro-comprimés à libération prolongée selon l'une quelconque des revendications précédentes.
